# EUROPEAN PATENT APPLICATION

(11) **EP 4 428 773 A1**
(43) Date of publication of application: **11.09.2024**
(21) Application number: 22889830.0
(22) Date of filing: 25.10.2022
(51) Int. Cl.: G06Q 10/04, G05B 19/418, G06N 20/00

(54) **FOOD CONTAMINATION PREDICTION DEVICE, INFERENCE DEVICE, MACHINE LEARNING DEVICE, FOOD CONTAMINATION PREDICTION METHOD, INFERENCE METHOD, AND MACHINE LEARNING METHOD**

(30) Priority: 02.11.2021 JP 2021179153
(71) Applicant: Toyo Seikan Group Holdings, Ltd., Shinagawa-ku, Tokyo 141-8627 (JP)
(72) Inventor: KUNIMASA Hidehiko, Tokyo 141-8627 (JP); TANABE Suguru, Tokyo 141-8627 (JP); FURUKAWA Satoshi, Tokyo 141-8627 (JP); OKAMURA Hiroshi, Tokyo 141-8627 (JP)
(74) Representative: Piticco, Lorena
(86) International application number: PCT/JP2022/039639
(87) International publication number: WO 2023/080010

(57) **Abstract**

[Object]

To provide a food contamination prediction device, an inference device, a machine learning device, a food contamination prediction method, an inference method, and a machine learning method that can easily predict the occurrence of a hazardous substance without directly inspecting the hazardous substance.

[Solution]

A food contamination prediction device (6) includes an information acquisition unit (600) that acquires environmental contamination indicator information including a detection status of an environmental contamination indicator different to a hazardous substance, and a prediction unit (601) configured to predict the occurrence status of the hazardous substance at the prediction point on the basis of the hazardous substance information, which is output when the environmental contamination indicator information acquired by the information acquisition unit (600) is input to a training model (12) learned by machine learning to learn a correlation between the environmental contamination indicator information including the detection status of the environmental contamination indicator at a training point and hazardous substance information including the occurrence status of the hazardous substance at the training point.

## Description

### Technical Field

The present disclosure relates to a food contamination prediction device, an inference device, a machine learning device, a food contamination prediction method, an inference method, and a machine learning method.

### Background Art

To ensure the safety of food produced in a food product production environment, each process until the food reaches the consumer is subjected to various food hygiene inspections. For example, Patent Document 1 discloses a hygiene measurement system including a hygiene information management computer, and a hygiene inspection device configured to inspect hygiene in individual food factories and send hygiene information, which is the result of the inspection, to the hygiene information management computer.

### Citation List

### Patent Literature

Patent Document 1: JP 2002-015007 B

### Summary

### Technical Problem

In the hygiene measurement system disclosed in Patent Document 1, a device configured to measure the number of bacteria in a food, for example, is used as the hygiene inspection device to perform inspection of the food. While the hygiene inspection device used in the hygiene inspection inspects types of bacteria classified as inspection targets, the hygiene inspection device cannot inspect types of bacteria not classified as inspection targets. Accordingly, in a case where a hazardous substance that may cause a food incident is bacteria not classified as an inspection target for the food hygiene inspection, the hygiene inspection device cannot perform an inspection of the hazardous substance. Additionally, even if the hazardous substance is a bacteria classified as an inspection target for the food hygiene inspection, the hygiene inspection device may take a long time to perform the inspection and it may take too long to prevent the food incident.

The present invention has been made in light of the above problem, and an object of the present invention is to provide a food contamination prediction device, an inference device, a machine learning device, a food contamination prediction method, an inference method, and a machine learning method that can easily predict the occurrence of a hazardous substance without directly inspecting the hazardous substance.

### Solution to Problem

To achieve the above object, a food contamination prediction device according to an aspect of the present invention is a food contamination prediction device that predict the occurrence of a hazardous substance that may cause a food incident, the food contamination prediction device comprising:
an information acquisition unit configured to acquire, at a prediction point, environmental contamination indicator information including a detection status of an environmental contamination indicator different to the hazardous substance; and
a prediction unit configured to predict the occurrence status of the hazardous substance at the prediction point on the basis of the hazardous substance information, the hazardous substance information being output when the environmental contamination indicator information acquired by the information acquisition unit is input to a training model
   learned by machine learning to learn a correlation between the environmental contamination indicator information including the detection status of the environmental contamination indicator at a training point and hazardous substance information including the occurrence status of the hazardous substance at the training point.

### Effects of Invention

With the food contamination prediction device according to an aspect of the present invention, the occurrence status of a hazardous substance can be easily predicted without directly inspecting the hazardous substance, on the basis of the hazardous substance information that is output when the environmental contamination indicator information, including the detection status of an environmental contamination indicator different from the hazardous substance, is input to a training model.

Problems, configurations, and effects other than those described above will be apparent in the embodiments of the present invention described below.

### Brief Description of Drawings

FIG. 1 is an overall configuration diagram illustrating an example of a food contamination prediction system 1.
FIG. 2 is an overall schematic diagram illustrating an example of a layout of a food factory 10.
FIG. 3 is a data configuration diagram illustrating an example of a process management database 40.
FIG. 4 is a data configuration diagram illustrating an example of a zone management database 41.
FIG. 5 is a data configuration diagram illustrating an example of a worker management database 42.
FIG. 6 is a data configuration diagram illustrating an example of an inspection database 43.
FIG. 7 is a hardware configuration diagram illustrating an example of a computer 900.
FIG. 8 is a block diagram illustrating an example of a machine learning device 5 according to a first embodiment.
FIG. 9 is a diagram illustrating an example of a training model 12 and training data 13 according to the first embodiment.
FIG. 10 is a flowchart illustrating an example of a machine learning method performed by the machine learning device 5.
FIG. 11 is a block diagram illustrating an example of a food contamination prediction device 6 according to the first embodiment.
FIG. 12 is a functional diagram illustrating an example of the food contamination prediction device 6 according to the first embodiment.
FIG. 13 is a flowchart illustrating an example of a food contamination prediction method performed by the food contamination prediction device 6.
FIG. 14 is a block diagram illustrating an example of a machine learning device 5a according to a second embodiment.
FIG. 15 is a diagram illustrating an example of a training model 12a and training data 13a according to the second embodiment.
FIG. 16 is a block diagram illustrating an example of a food contamination prediction device 6a according to the second embodiment.
FIG. 17 is a functional diagram illustrating an example of the food contamination prediction device 6a according to the second embodiment.
FIG. 18 is a block diagram illustrating an example of a machine learning device 5b according to a third embodiment.
FIG. 19 is a diagram illustrating an example of a training model 12b and training data 13b according to the third embodiment.
FIG. 20 is a block diagram illustrating an example of a food contamination prediction device 6b according to the third embodiment.
FIG. 21 is a functional diagram illustrating an example of the food contamination prediction device 6b according to the third embodiment.
FIG. 22 is a block diagram illustrating an example of a machine learning device 5c according to a fourth embodiment.
FIG. 23 is a diagram illustrating an example of a training model 12c and training data 13c according to the fourth embodiment.
FIG. 24 is a block diagram illustrating an example of a food contamination prediction device 6c according to the fourth embodiment.
FIG. 25 is a functional diagram illustrating an example of the food contamination prediction device 6c according to the fourth embodiment.

### Description of Embodiments

Hereinafter, embodiments for carrying out the present invention will be described with reference to the drawings. Herein, a scope necessary for explaining the invention to achieve the object of the present invention is shown schematically, and the explanation shall be mainly to the extent necessary for the explanation of the relevant part of the invention. Any omitted explanations are based on the known art.

### First Embodiment

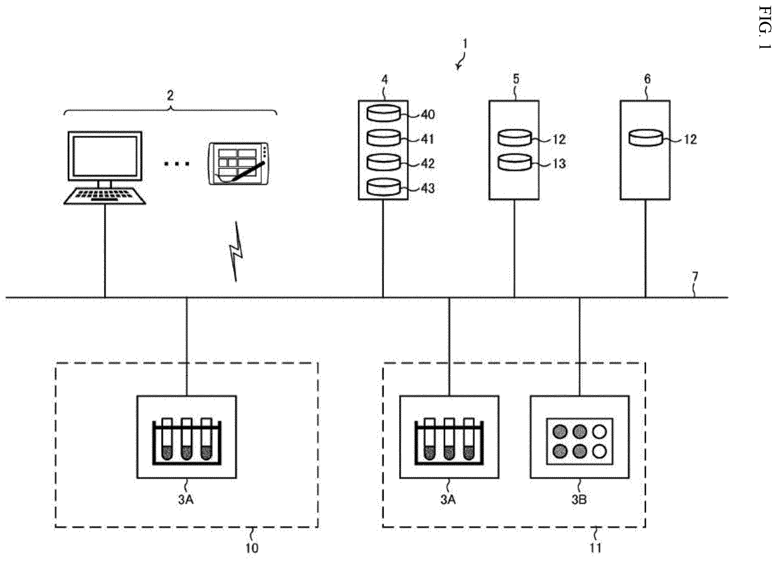

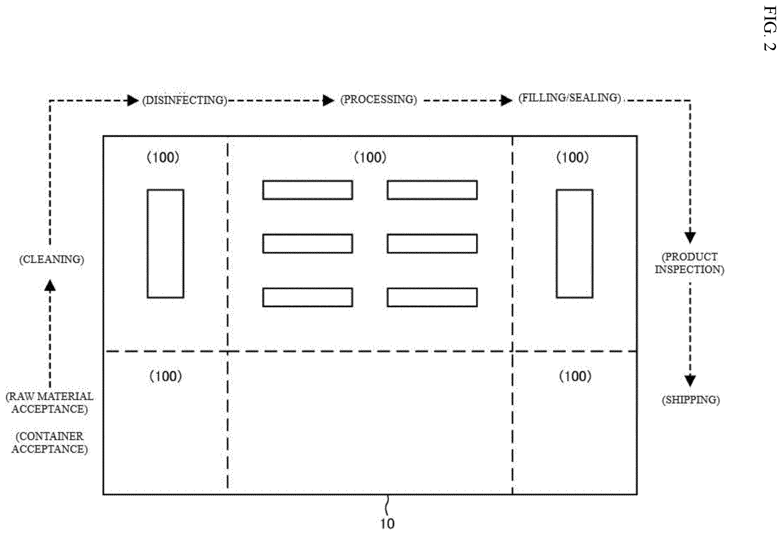

FIG. 1 is an overall configuration diagram illustrating an example of a food contamination prediction system 1. FIG. 2 is an overall schematic diagram illustrating an example of a layout of a food factory 10.

The food contamination prediction system 1 according to the present embodiment is a system applied in the food factory 10, which is a food production environment in which food is produced from raw ingredients to semi-products to products. The food contamination prediction system 1 functions as a system that manages the occurrence of hazardous substances that may cause food incidents in the food factory 10 on the basis of the detection status of environmental contamination indicators, which are different from hazardous substances. The raw ingredients are, for example, agricultural products, livestock products, and marine products, and the products manufactured in the food factory 10 are, for example, any food products such as fresh food, processed food, and beverages.

An environmental contamination indicator is an indicator used to evaluate the state of contamination in the food production environment. The environmental contamination indicator can be evaluated by detecting a situation in which a certain factor is present in the food production environment. Examples of the specific factor include bacteria, viruses, allergens, viroids, and molds. Specific examples of the bacteria include general bacteria, coliform bacteria, Escherichia coli (E. coli), enterococci, heat-resistant spore bacteria, clostridia, staphylococci, and Vibrio spp. Specific examples of the virus include norovirus and sapovirus. Specific examples of the allergen include egg-derived protein, milk-derived protein, wheat-derived protein, shrimp-derived protein, and crab-derived protein. Specific examples of the viroid include potato or semo viroids and tomato degreening and atrophy viroids. Specific examples of the mold include black mold, blue mold, and stink mold. Note that the environmental contamination indicator is not particularly limited and may be any indicator that indicates the state of contamination of the food production environment.

The hazardous substance is a substance that can cause a food incident. Examples of the hazardous substance include Salmonella, Vibrio parahaemolyticus, Staphylococcus aureus, Listeria monocytogenes, Campylobacter, enterohemorrhagic E. coli (e.g., 0157), Bacillus cereus, and Bacillus welsh. The hazardous substance is not limited to the above examples and may be any substance that can cause a food incident.

As illustrated in FIG. 2, in the food factory 10, a food production process consisting of multiple processes such as, for example, receiving raw materials, receiving containers, washing, sterilizing, processing (which may include multiple processing stages), filling, sealing, product inspection, and shipping are performed sequentially. The food factory 10 has defined zones 100 for performing each process. Each zone 100 is equipped with, for example, food washing equipment, food sterilization equipment, food processing equipment, food conveying equipment, cooking utensils, worktables, and other equipment and devices. Each zone 100 is managed according to individual attributes referred to as contaminated work area, semi-clean work area, and clean work area.

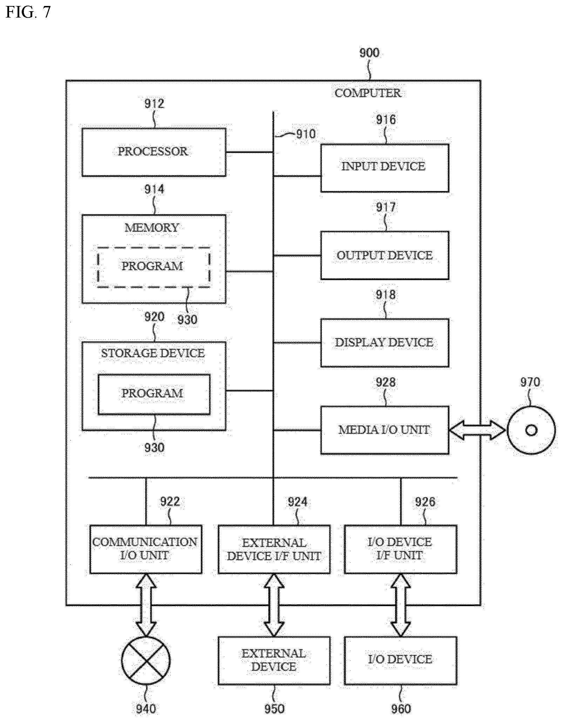

As illustrated in FIG. 1, the food production management system 1 includes as main components a worker terminal device 2, an environmental contamination indicator inspection device 3A, a hazardous substance inspection device 3B, a production control device 4, a machine learning device 5, and a food contamination prediction device 6. Each device 2 to 6 consists of a general-purpose or dedicated computer (see FIG. 7 below), for example, and is connected to a wired or wireless network 7 to send/receive various data to/from each other. The number of each device 2 to 6 and the connection configuration of the network 7 are not limited to the example illustrated in FIG. 1 and may be changed as needed.

The worker terminal device 2 is a terminal device used by a worker (e.g., a process worker, an inspection worker, or a factory manager) working in the food factory 10, and may be a stationary device or a portable device. For example, the worker terminal device 2 accepts various input operations via a display screen such as an application program or a web browser, and a reader/writer, and displays various information via the display screen.

The environmental contamination indicator inspection device 3A is a device configured to detect environmental contamination indicators contained in samples. The environmental contamination indicator inspection device 3A is used, for example, to inspect environmental contamination indicators adhering to equipment and doorknobs used in the food factory 10, to inspect environmental contamination indicators adhering to food products (e.g., raw materials, semi-finished products, or finished products) handled in the food factory 10, to inspect environmental contamination indicators adhering to workers' bodies and work clothes, and to inspect airborne environmental contamination indicators in the food factory 10. The environmental contamination indicator inspection device 3A is also used to inspect environmental contamination indicators in food contamination analysis tests conducted in a laboratory 11 that simulates the food factory 10 as a test environment. Note that, although the environmental contamination indicator inspection device 3A uses one or more types of the environmental contamination indicators as inspection targets, multiple types of environmental contamination indicator inspection devices 3A using different inspection targets and inspection methods may be used.

The environmental contamination indicator inspection device 3A is, for example, an inspection kit or a measuring instrument, and inspects the detection status of the environmental contamination indicators. In a case where the environmental contamination indicator inspection device 3A is configured to output the detection status of the environmental contamination indicators as data, environmental contamination indicator detection information indicating the detection status of the environmental contamination indicators is transmitted to, for example, the production management device 4 or the food contamination prediction device 6. In a case where the environmental contamination indicator inspection device 3A does not have a function to transmit the environmental contamination indicator detection information as described above, the inspection worker performs an input operation to input the detection status of the environmental contamination indicators to the worker terminal device 2, and the worker terminal device 2 transmits the environmental contamination indicator detection information based on that input operation.

The hazardous substance inspection device 3B is a device configured to detect a hazardous substance contained in a sample, and is used to inspect for hazardous substances in food contamination analysis tests. Note that, while the hazardous substance inspection device 3B is configured to inspect for one or more types of hazardous substances as inspection targets, a plurality of types of hazardous substance inspection devices 3B each using different inspection targets and inspection methods may be used. The hazardous substance inspection device 3B may also be used for inspection hazardous substances in the food factory 10.

Similar to the environmental contamination indicator inspection device 3A, the hazardous substance inspection device 3B consists of components such as an inspection kit and measuring instruments and is configured to inspect for a hazardous substance. In a case where the hazardous substance inspection device 3B is configured to output data related to the occurrence status of a hazardous substance, hazardous substance occurrence information indicating the occurrence status of the hazardous substance is transmitted to, for example, the machine learning device 5. In a case where the hazardous substance inspection device 3B is not equipped with a function of transmitting hazardous substance occurrence information as described above, the inspection worker performs an input operation to input the occurrence status of a hazardous substance to the worker terminal device 2, and the worker terminal device 2 transmits the hazardous substance occurrence information based on that input operation.

Note that the environmental contamination indicator inspection device 3A and the hazardous substance inspection device 3B may employ any inspection method among a morphological inspection, biochemical inspection, serological inspection, and genetic inspection, or may employ a combination of these methods.

The production management device 4 includes a process management database 40 for managing the implementation status of each process in the food manufacturing process, a zone management database 41 for managing the attributes, space conditions, cleaning conditions, and the like of each zone 100 in the food factory 10, a worker management database 42 for managing the work status of workers working in each zone 100, and an inspection database 43 for managing the inspection results of the environmental contamination indicators performed by the environmental contamination indicator inspection device 3A. Details of each database 40 to 43 will be given later. Note that, the production management device 4 may be a device that manages a plurality of food factories 10.

The machine learning device 5 is a device that operates as the subject in the learning phase of machine learning. For example, the machine learning device 5 acquires test results of a food contamination analysis test performed in a laboratory 11 as training data 13, and generates a training model 12 to be used by the food contamination prediction device 6 by machine learning. The training model 12 after training is provided to the food contamination prediction device 12 via a network 7 or a storage medium.

The food contamination analysis test is performed under various test conditions to replicate various potential food production environments in the food factory 10. The environmental contamination indicator inspection device 3A and the hazardous substance inspection device 3B is used for each of a plurality of samples each sampled at a specific point (study point) under each test condition. Thereby, inspection results of the environmental contamination indicator inspection device 3A (detection status of environmental contamination indicators) and inspection results of the hazardous substance inspection device 3B (occurrence of hazardous substances) are obtained. Note that, while the food contamination analysis test is generally conducted in the laboratory 11 set up at a different location from the food factory 10, the food contamination analysis test may be conducted using the food factory 10.

The food contamination prediction device 6 is a device that operates as the subject of the inference phase of machine learning. The food contamination prediction device 6 uses the training model 12 generated by the machine learning device 5 to predict the occurrence of hazardous substances at a specific point (prediction point) in the food factory 10 on the basis of the detection status of the environmental contamination indicators for each sample sampled at that point. The hazardous substance generation information indicating the occurrence of a hazardous substance as the prediction result is, for example, transmitted to the worker terminal device 2 and presented to an inspection worker or factory administrator. Additionally, the hazardous substance generation information is transmitted to the production management device 4 and stored in the inspection database 43.

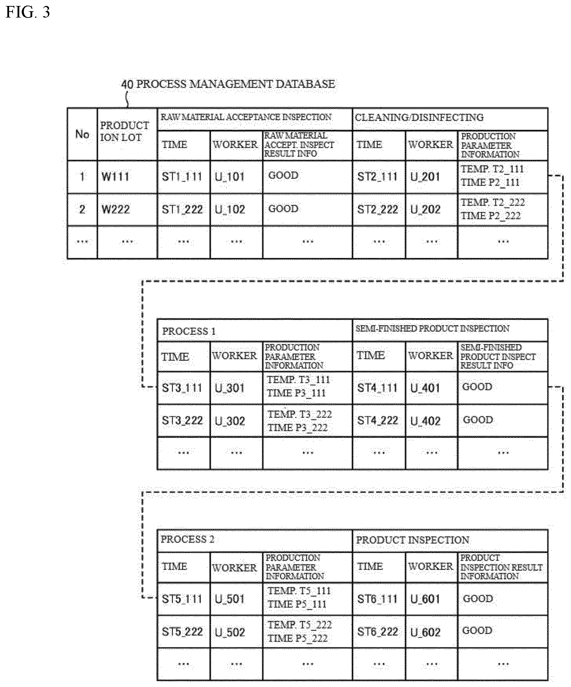

FIG. 3 is a data configuration diagram illustrating an example of the process management database 40. The process management database 40 is a database that manages the implementation status of each process for each production lot.

The production management device 4 receives production parameter information indicating production parameters from, for example, food washing equipment, food sterilization equipment, and food processing equipment used in a washing process, a sterilization process, and a processing process, respectively, as needed, and registers the production parameter information in the process management database 40. When inspection processes for raw materials, semi-finished products, and finished products are conducted by an inspection worker, the production management device 4 receives raw material acceptance inspection result information and semi-finished product inspection result information indicating the results of each inspection process from the worker terminal device 2 used by the process worker as needed, and registers this information in the process management database 40.

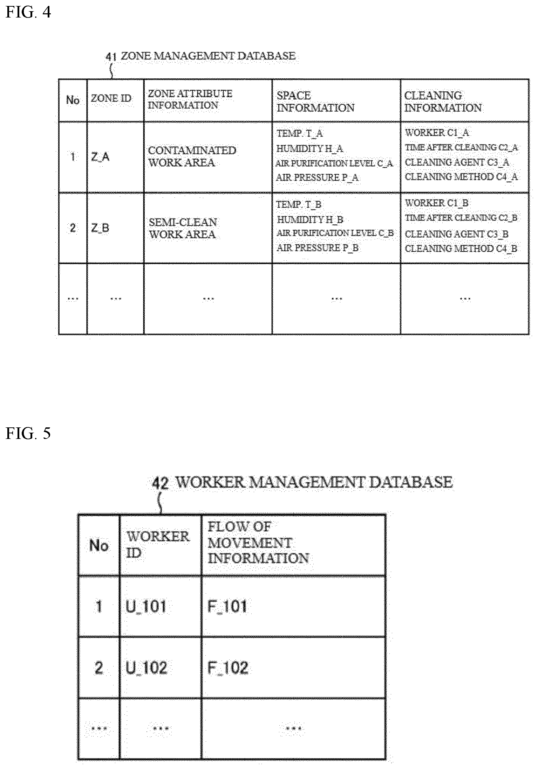

FIG. 4 is a data configuration diagram illustrating an example of the zone management database 41. The zone management database 41 is a database that manages the attributes, space status, and cleaning status of each zone 100.

The production management device 4 receives zone attribute information indicating the attributes of each zone 100 (contaminated work zone, semi-clean work zone, and clean work zone) from the worker terminal device 2 used by the factory administrator, and registers the zone attribute information in the zone management database 41. The production management device 4 receives, as needed, space information indicating the space status (e.g., temperature, humidity, air purification level, and air pressure) of each zone 100 from an environmental control device group (e.g., air conditioner, ventilator, and air purifier) used to control the space status of each zone 100 in the food factory 10, and registered the space information in the zone management database 41. In this case, the space information is registered as time series data in the zone management database 41 each time the temperature, humidity, air purification level, air pressure and the like are measured at predetermined measurement intervals. When a cleaning task is performed by a process worker in each zone 100, the production management device 4 receives the cleaning information indicating the cleaning status of each zone 100 from the worker terminal device 2 used by that process worker and registers the cleaning information in the zone management database 41.

FIG. 5 is a data configuration diagram illustrating an example of the worker management database 42. The worker management database 42 is a database that manages a worker ID and flow of movement information for each worker.

The production management device 4 receives position information of a worker from the worker terminal device 2 used by the worker or an entry/exit management system (not illustrated) as needed, and registers flow of movement information indicating the flow of movement (movement trajectory) of the worker in the worker management database 42. The worker ID is, for example, used as information for identifying a worker who performed a process registered in the process management database 40 or performed a cleaning task registered in the zone management device 41. Thus, it is possible to identify the workers' flow of movement when each process or cleaning operation is performed by referring to the flow of movement information and the process management database 40 or the zone management database 41.

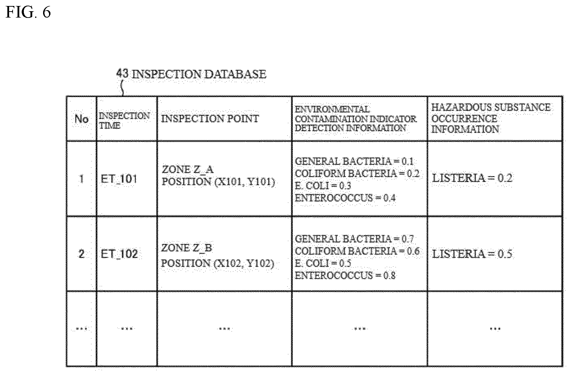

FIG. 6 is a data configuration diagram illustrating an example of the inspection database 43. The inspection database 43 is a database that manages the inspection time, inspection point, environmental contamination indicator detection information, and hazardous substance occurrence information for the inspection operation of each environmental contamination indicator by the environmental contamination indicator inspection device 3A.

When inspection work with respect to the environmental contamination indicator is performed by an inspection worker, the production management device 4 receives the environmental contamination indicator detection information from the worker terminal device 2 used by the inspection worker or the environmental contamination indicator inspection device 3A as needed, and registers the environmental contamination indicator detection information in the inspection database 43. At this time, the inspection time and the inspection point (e.g., the zone 100, positional coordinates in the zone 100) acquired at the time of sampling the sample are also registered. Additionally, the production management device 4 receives the hazardous substance occurrence information (predicted result) relating to the environmental contamination indicator detection information acquired by the food contamination prediction device 6 from the food contamination prediction device 6, as needed, and registers the hazardous substance occurrence information (predicted result) in the inspection database 43. Note that, in a case where the hazardous substance inspection device 3B is used to inspect for a hazardous substance in a food factory, and the production management device 4 receives the hazardous substance occurrence information (inspection result) acquired by the hazardous substance inspection device 3B from the worker terminal device 2 or the hazardous substance inspection device 3B, the hazardous substance occurrence information (inspection result) may be registered in the inspection database 43. In this case, the predicted result and inspection result of the hazardous substance occurrence information is registered in the inspection database 43 in a manner that the predicted result and inspection result can be distinguished from each other.

### Hardware Configuration of Each Device

FIG. 7 is a hardware configuration diagram illustrating an example of a computer 900. The devices 2 to 6 of the food production management system 1 are each configured by a general or specialist computer 900.

As illustrated in FIG. 7, the computer 900 includes, as main components, a bus 910, a processor 912, a memory 914, an input device 916, an output device 917, a display device 918, a storage device 920, a communication interface (I/F) 922, an external device I/F 924, an input/output (I/O) device I/F 926, and a media I/O 928. Note that, the above-described components may be omitted as necessary depending on how the computer 900 is to be used.

The processor 912 is configured by one or more arithmetic processing units (e.g., a central processing unit (CPU), a micro-processing unit (MPU), a digital signal processor (DSP), or a graphics processing unit (GPU)), and operates as a control unit that controls the entire computer 900. The memory 914 stores various data and programs 930 and consists of, for example, a volatile memory (e.g., a DRAM or an SRAM) that functions as the main memory, a non-volatile memory (ROM), and a flash memory.

The input device 916 is configured by, for example, a keyboard, a mouse, a numeric keypad, or an electronic pen and functions as an input unit. The output device 917 is configured by, for example, a sound (voice) output device or a vibration device and functions as an output unit. The display device 918 is configured by, for example, a liquid crystal display, an organic EL display, electronic paper or a projector and functions as an output unit. The input device 916 and the display device 918 may be configured as an integral device, such as a touch panel display. The storage device 920 is configured by, for example, an HDD or a solid state drive (SSD) and functions as a storage unit. The storage device 920 stores various data necessary for executing the operating system and programs 930.

The communication I/F 922 is wired or wirelessly connected to a network 940 such as the Internet or an intranet (which may be the same as the network 7 in FIG. 1) and functions as a communication unit that transmits/receives data to/from another computer in accordance with a predetermined communication standard. The external device I/F 924 is wired or wirelessly connected to an external device 950 such as a camera, a printer, a scanner, or a reader/writer, and functions as a communication unit that transmits/receives data to/from the external device 950 in accordance with a predetermined communication standard. The I/O device I/F 926 is connected to various sensors and an I/O device 960, such as an actuator, and functions as a communication unit that transmits/receives various signals and data, such as detection signals obtained by the sensors and signals for controlling the actuator, to/from the I/O device 960. The media I/O unit 928 is configured by a drive device, such as a DVD drive or a CD drive, and reads/writes data to/from a medium (non-transient storage medium) 970 such as a DVD or a CD.

In the computer 900 having the above configuration, the processor 912 calls out the program 930 stored in the storage device 920 to the memory 914 and executes the program 930 to control various units of the computer 900 via the bus 910. Note that, the program 930 may be stored in the memory 914 instead of in the storage device 920. The program 930 may be recorded on the medium 970 in an installable or executable file format and provided to the computer 900 via the media I/O unit 928. The program 930 may be provided to the computer 900 by being downloaded over the network 940 via the communication I/F unit 922. The computer 900 may also have various functions realized when the processor 912 executes the program 930 in the form of hardware, such as an FPGA or an ASIC.

The computer 900 is any form of electronic device configured by, for example, a stationary computer or a portable computer. The computer 900 may be a client computer, a server computer, or a cloud computer. The computer 900 may be applied to devices other than the devices 2 to 6.

### Machine Learning Device 5

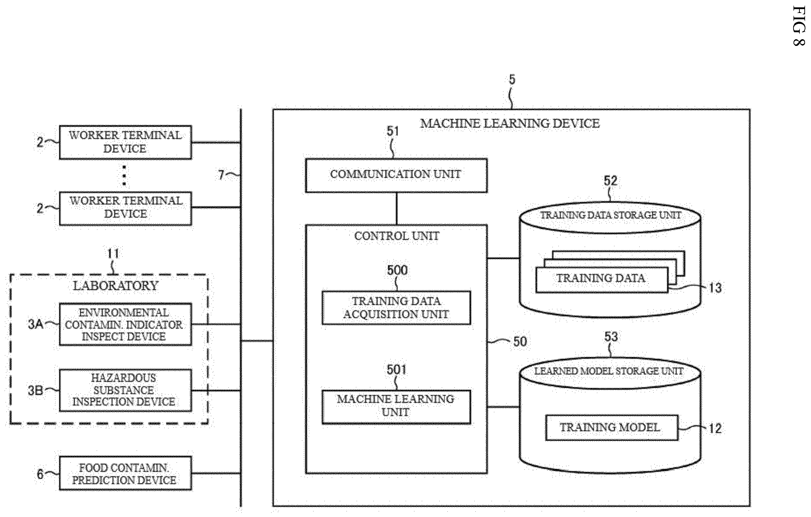

FIG. 8 is a block diagram illustrating an example of the machine learning device 5 according to a first embodiment. The machine learning device 5 includes a control unit 50, a communication unit 51, a training data storage unit 52, and a learned model storage unit 53.

The control unit 50 functions as a training data acquisition unit 500 and a machine learning unit 501. The communication unit 51 is connected to an external device (e.g., the worker device terminal 2, the environmental contamination indicator inspection device 3A, the hazardous substance inspection device 3B, and the food contamination prediction device 6) via the network 7, and functions as a communication interface that transmits/receives various types of data.

The training data acquisition unit 500 is connected to an external device via the communication unit 51 and the network 7 and acquires the training data 13, which consists of the environmental contamination indicator information serving as input data and the hazardous substance information serving as output data. The training data 13 is data used as teacher data (training data), validation data, and test data in supervised learning. The hazardous substance information is the data used as the correct answer labels in supervised learning.

The training data storage unit 52 is a database that stores multiple sets of the training data 13 acquired by the training data acquisition unit 500. The specific configuration of the database comprising the training data storage unit 52 may be configured as needed.

The machine learning unit 501 performs machine learning using the multiple sets of training data 13 stored in the training data storage unit 52. In other words, the machine learning unit 501 inputs the multiple sets of training data 13 to the training model 12 and causes the training model 12 to learn the correlation between the environmental contamination indicator information and the hazardous substance information in the training data 13, thereby generating a learned training model 12.

The learned model storage unit 53 is a database that stores the learned training model 12 (specifically, an adjusted set of weight parameters) generated by the machine learning unit 501. The learned training model 12 stored in the learned model storage unit 53 is provided to a real system (e.g., the food contamination prediction device 6) via the network 7 or a storage medium. Note that, in FIG. 8, while the training data storage unit 52 and the learned model storage unit 53 are illustrated as separate storage units, these units may be configured as a single storage unit.

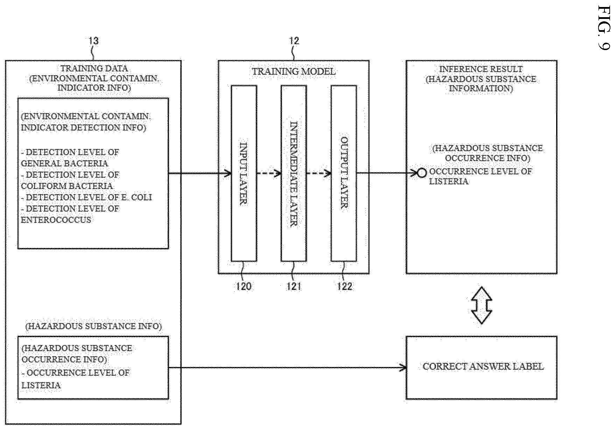

FIG. 9 is a diagram illustrating an example of the training model 12 and the training data 13 according to the first embodiment. The training data 13 used in machine learning of the training model 12 is made up of the environmental contamination indicator information and the hazardous substance information.

The environmental contamination indicator information of the training data 13 includes environmental contamination indicator detection information indicating the detection status of environmental contamination indicators at a training point. The detection status of the environmental contamination indicator is the detection status of one or more types of the environmental contamination indicators and is expressed, for example, as the presence or absence of detection, number of detections, or detection level. The detection level may be a step value or a continuous value. In the case of a continuous value, the detection level may be a value normalized to a predetermined range (e.g., 0 to 1).

In the present embodiment, a case will be described in which the detection status of the environmental contamination indicator included in the environmental contamination indicator information is the detection level (normalized in the range from 0 to 1) of four types of environmental contamination indicators consisting of general bacteria, coliforms, E. coli, and enterococci. Note that, the detection status of another environmental contamination indicator may also be used as the environmental contamination indicator information. In that case, the data configuration of the input data for the training model 12 and the training data 13 may be changed as required.

The hazardous substance information in the training data 13 includes hazardous substance occurrence information indicating the occurrence status of a hazardous substance at the training point corresponding to the environmental contamination indicator detection information. The occurrence status of the hazardous substance refers to the occurrence status of one or more types of the hazardous substances and is expressed, for example, as the presence or absence of occurrence, the number of occurrences, or the occurrence level. The occurrence level may be a step value or a continuous value. In the case of a continuous value, the occurrence level may be a value normalized to a predetermined range (e.g., 0 to 1).

In the present embodiment, a case will be described in which the occurrence status of the hazardous substance included in the hazardous substance information is the occurrence level (normalized in the range from 0 to 1) of listeria. Note that, the occurrence status of another hazardous substance may also be used as the hazardous substance information. In that case, the data configuration of the output data for the training model 12 and the training data 13 may be changed as required.

When the food contamination analysis test is conducted, the training data acquisition unit 500 receives the environmental contamination indicator detection information acquired by the environmental contamination indicator inspection device 3A from the worker terminal device 2 or the environmental contamination indicator inspection device 3A, and receives the hazardous substance occurrence information acquired by the hazardous substance inspection device 3B from the worker terminal device 2 or the hazardous substance inspection device 3B, thereby acquiring the training data 13.

The training model 12 is, for example, a model with a neural network structure and includes an input layer 120, an intermediate layer 121, and an output layer 122. Synapses (not illustrated) connecting each neuron are stretched between each layer, and a weight is associated with each synapse. A set of weight parameters consisting of the weights of each synapse is adjusted by machine learning.

The input layer 120 includes neurons in a quantity corresponding to the environmental contamination indicator information as input data. The value of each environmental contamination indicator information is input to each neuron. The output layer 122 includes neurons in a quantity corresponding to the hazardous substance information as output data. The prediction result (inference result) of the hazardous substance information for the environmental contamination indicator information is output as output data. In a case where the training model 12 consists of a regression model, each piece of the hazardous substance information is output as a numerical value normalized to a predetermined range (e.g., 0 to 1). In a case where the training model 12 consists of a classification model, each piece of the hazardous substance information is output as a score (probability) for each class, as a value normalized to a predetermined range (e.g., 0 to 1).

### Machine Learning Method

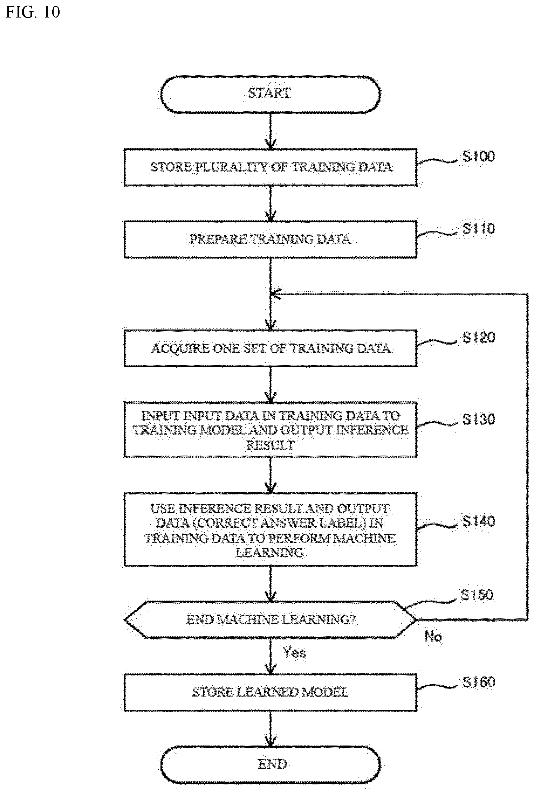

FIG. 10 is a flowchart illustrating an example of a machine learning method performed by the machine learning device 5.

First, in step S100, the training data acquisition unit 500 acquires the training data 13 in a desired number as preparation for starting machine learning. The acquired training data 13 is stored in the training data storage unit 52. The number of the training data 13 acquired in this step may be set in consideration of the inference accuracy required of the final training model 12.

Subsequently, in step S110, the machine learning unit 501 prepares the training model 12 in a state before training to start machine learning. The training model 12 in a state before training prepared in this step is the neural network model illustrated in FIG. 9, with the weight of each synapse set to an initial value.

Subsequently, in step S120, the machine learning unit 501 randomly acquires one set of the training data 13 from multiple sets of the training data 13 stored in the training data storage unit 52.

Subsequently, in step S130, the machine learning unit 501 inputs the environmental contamination indicator information (input data) included in the set of training data 13 into the input layer 120 of the prepared training model 12 before training (or during training). As a result, the hazardous substance information (output data) is output from the output layer 122 of the training model 12 as an inference result. This output data is generated by the training model 12 before training (or during training). Thus, in the state before training (or during training), the output data output as an inference result indicates information different to the hazardous substance information (correct answer label) included in the training data 13.

Subsequently, in step S140, the machine learning unit 501 performs machine learning by comparing the hazardous substance information (correct answer label) included in the set of training data 13 acquired in step S 120 and the hazardous substance information (output data) output as the inference result from the output layer in step S130 and performing processing of adjusting the weight of each synapse. Thereby, the machine learning unit 501 causes the training model 12 to learn the correlation between the environmental contamination indicator information and the hazardous substance information.

Subsequently, in step S150, the machine learning unit 501 determines whether a predetermined learning termination condition is satisfied on the basis of, for example, an evaluation value of an error function based on the hazardous substance information (correct answer label) in the training data 13 and the hazardous substance information (output data) output as the inference result, or the remaining number of unlearned pieces of training data 13 stored in the training data storage unit 52.

In step S150, in a case where the machine learning unit 501 determines that the learning termination condition is not satisfied and that machine learning should continue (No in step S150), the processing returns to step S120 and the machine learning unit 501 performs, a plurality of times, the processes of steps S120 to S140 on the training model 12 during training using the unlearned training data 13. On the other hand, in step S150, in a case where the machine learning unit 501 determines that the learning termination condition is satisfied and to terminate the machine learning (Yes in step S150), the processing proceeds to step S160.

Subsequently, in step S160, the machine learning unit 501 stores the learned training model 12 (adjusted weight parameter group) generated by adjusting the weights associated with each synapse in the learned model storage unit 53, and ends the series of machine learning methods illustrated in FIG. 10. In the machine learning method, step S 100 corresponds to a training data storage process, steps S 110 to S 150 correspond to a machine learning process, and step S160 corresponds to a learned model storage process.

As described above, with the food contamination prediction device 6 and the food contamination prediction method according to the present embodiment, there can be provided a training model 12 that can predict (infer) hazardous substance information related to environmental contamination indicator information from the environmental contamination indicator information including environmental contamination indicator detection information.

### Food Contamination Prediction Device 6

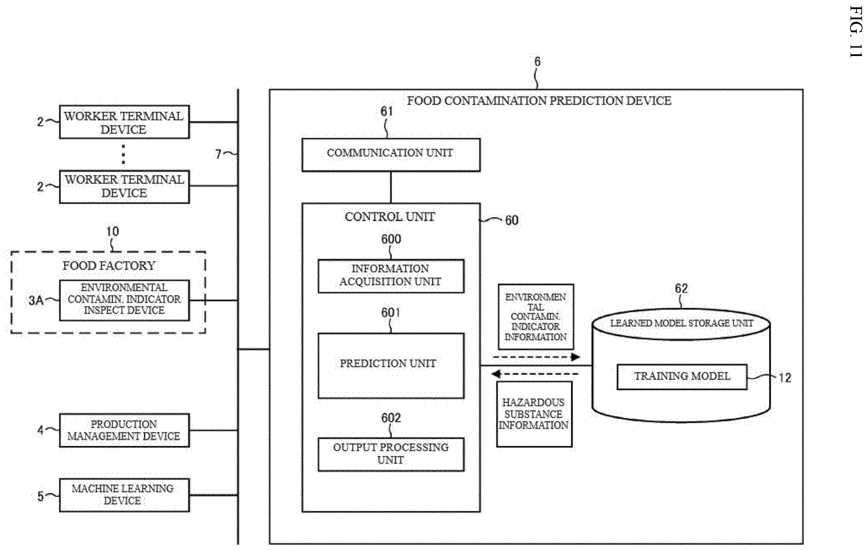

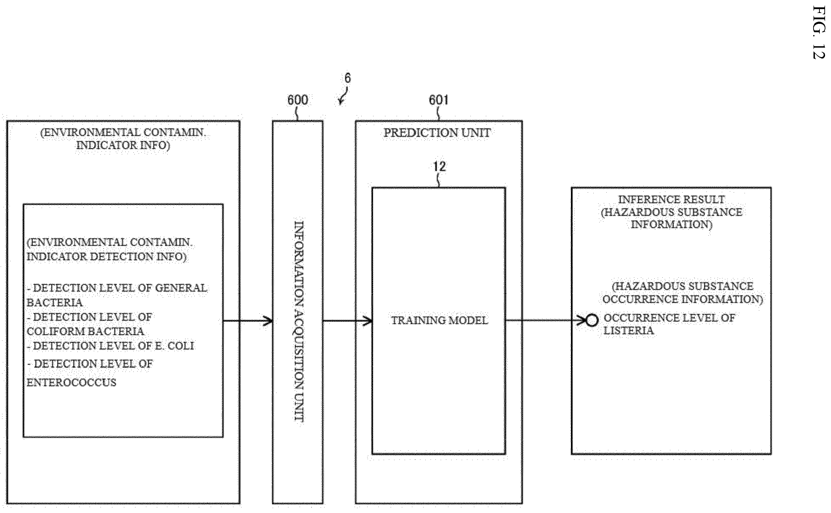

FIG. 11 is a block diagram illustrating an example of the food contamination prediction device 6 according to the first embodiment. FIG. 12 is a functional diagram illustrating an example of the food contamination prediction device 6 according to the first embodiment. The food contamination prediction device 6 includes a control unit 60, a communication unit 61, and a learned model storage unit 62.

The control unit 60 functions as an information acquisition unit 600, a prediction unit 601 and an output processing unit 602. The communication unit 61 is connected to an external device (e.g., the worker terminal device 2, the environmental contamination indicator inspection device 3A, the production management device 4, or the machine learning device 5) and functions as a communication interface that transmits/receives various types of data.

The information acquisition unit 600 is connected to an external device via the communication device 61 and the network 7 and acquires the environmental contamination indicator information including the environmental contamination indicator detection information indicating the detection status of an environmental contamination indicator at a predicted point.

For example, while each process of the food production process is being performed, the information acquisition unit 600 acquires the environmental contamination indicator information by receiving the environmental contamination indicator detection information from the worker terminal device 2 or the environmental contamination indicator inspection device 3A as an inspection result at the time the environmental contamination indicator inspection device 3A conducts the environmental contamination indicator inspection at a specific point (predicted point) in the food factory 10. In addition, in a case where the inspection result of the environmental contamination indicator is registered in the inspection database 43, the information acquisition unit 600 acquires the environmental contamination indicator information by receiving the environmental contamination indicator detection information from the production management device 4.

As described above, the prediction unit 601 predicts the occurrence status of a hazardous substance at the predicted point on the basis of output hazardous substance information by inputting the environmental contamination indicator information at the predicted point acquired by the information acquisition unit 600 to the training model 12 as input data.

The learned model storage unit 62 is a database that stores the learned training model 12 used by the prediction unit 601. Note that, the number of the training models 12 stored in the learned model storage unit 62 is not limited to one, and a plurality of learned models having different conditions may be stored and used selectively. Examples of the different conditions include the machine learning method, the type of environmental contamination indicator inspection device 3A (inspection target and inspection method), the type of hazardous substance inspection device 3B (inspection target and inspection method), the type of data included in the environmental contamination indicator information, and the type of data included in the hazardous substance information. Additionally, the learned model storage unit 62 may be substituted with a storage unit of an external computer (e.g., a server computer or a cloud computer). In this case, the prediction unit 601 need only access the external computer.

The output processing unit 602 performs output processing for outputting the hazardous substance information generated by the prediction unit 601. For example, the output processing unit 602 may transmit the hazardous substance information to the worker terminal device 2, so that a display screen based on the hazardous substance information is displayed on the worker terminal device 2. Alternatively, the output processing unit 602 may transmit the hazardous substance information to the production management device 4, so that the hazardous substance information is registered in the inspection database 43.

### Food Contamination Prediction Method

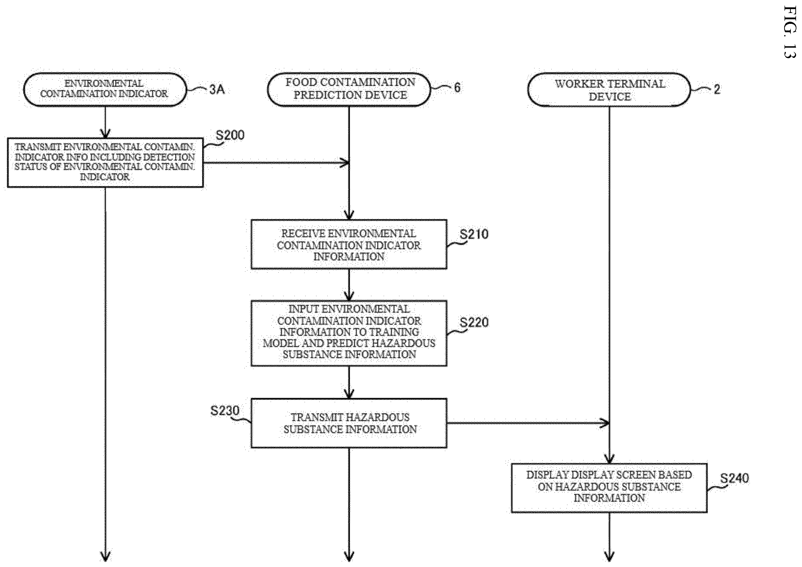

FIG. 13 is a flowchart illustrating an example of a food contamination prediction method performed by the food contamination prediction device 6. The following description will deal with an operation example of the food contamination prediction device 6 in which the inspection worker uses the environmental contamination indicator inspection device 3A to inspect for the environmental contamination indicator.

First, in step S200, when the inspection worker inspects for the environmental contamination indicator at the predicted point by using the environmental contamination indicator inspection device 3A, the environmental contamination indicator inspection device 3A transmits the environmental contamination indicator detection information being the detection result to the food contamination prediction device 6.

Subsequently, in step S210, the information acquisition unit 600 of the food contamination prediction device 6 receives the environmental contamination indicator detection information transmitted in step S200 and acquires the environmental contamination indicator information including the detection status of the environmental contamination indicator at the predicted point.

Subsequently, in step S220, the prediction unit 601 inputs the environmental contamination indicator information acquired in step S210 to the training model 12, generates hazardous substance information related to the environmental contamination indicator information as output data, and predicts the occurrence status of a hazardous substance at the predicted point.

Subsequently, in step S230, as output processing for outputting the hazardous substance information generated in step S220, the output processing unit 602 transmits the hazardous substance information to the worker terminal device 2 used by the inspection worker or factory administrator. Note that, the hazardous substance information may also be transmitted to the production management device 4 in addition to or in place of the worker terminal device 2.

Subsequently, in step S240, the worker terminal device 2 receives the hazardous substance information transmitted in step S230 and displays a display screen on the basis of the hazardous substance information, thereby presenting the occurrence status of the hazardous substance at the predicted point to the inspection worker or factory administrator. In the above-described food contamination prediction method, step S210 corresponds to an information acquisition step, step S220 corresponds to a prediction step, and step S230 corresponds to an output processing step.

As described above, with the food contamination prediction device 6 and the food contamination prediction method according to the present invention, since the occurrence status of the hazardous substance is predicted on the basis of the hazardous substance information that is output when the environmental contamination indicator information, including the detection status of an environmental contamination indicator different from the hazardous substance, is input to the training model 12, the occurrence status of the hazardous substance can be easily predicted without directly inspecting the hazardous substance.

### Second Embodiment

A second embodiment differs from the first embodiment in that the environmental contamination indicator information includes food production environment information indicating the status of a food production environment including a predicted point and a training point, in addition to the environmental contamination indicator detection information. The following description will focus on differences to the first embodiment, namely, a machine learning device 5a and a food contamination prediction device 6a according to the second embodiment.

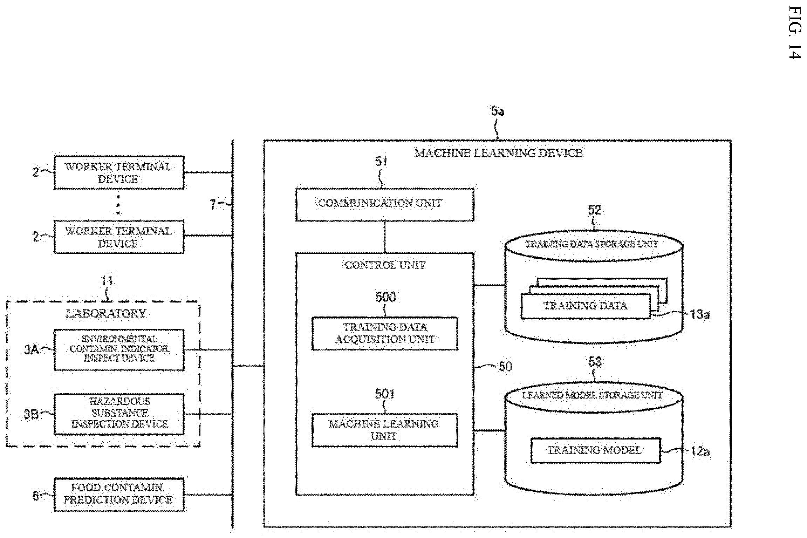

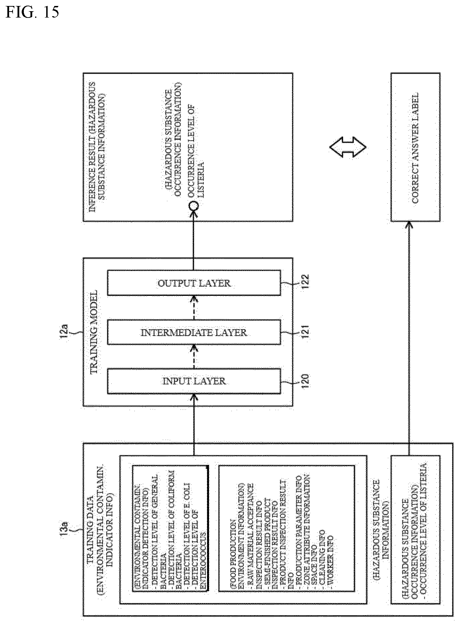

FIG. 14 is a block diagram illustrating an example of the machine learning device 5a according to the second embodiment. FIG. 15 is a diagram illustrating an example of a training model 12a and training data 13a according to the second embodiment. The training data 13a is used in machine learning for the training model 12a.

The environmental contamination indicator information in the training data 13a includes environmental contamination indicator detection information at the training point and food production environment information that indicates the status of the food production environment including the learning point. The environmental contamination indicator information includes, as food production environment information that affects the occurrence status of the hazardous substance, raw material acceptance inspection result information, semi-finished product inspection result information, product inspection result information, production parameter information, zone attribute information, space information, cleaning information, and worker information. Each type of information included in the food production environment information corresponds to the information registered in the process management database 40 (FIG. 3), the zone management database 41 (FIG. 4), and the worker management database 42 (FIG. 5). The environmental contamination indicator information may include information other than that described above, as long as the information indicates the status of the food production environment. Note that, the hazardous substance information of the training data 13a is the same as in the first embodiment, and thus explanation is omitted.

The raw material acceptance inspection result information indicates an inspection result of an acceptance inspection conducted for raw materials and indicates, for example, pass or fail. The semi-finished product inspection result information indicates an inspection result of an inspection conducted on a semi-finished product and indicates, for example, pass or fail. The finished product inspection result information indicates an inspection result of an inspection conducted on a finished product and indicates, for example, pass or fail.

The production parameter information indicates production parameters for the food production process and includes, for example, washing temperature and washing time of food washing equipment, sterilization temperature and sterilization time of food sterilization equipment, and heating temperature and heating time of food processing equipment.

The zone attribute information indicates the attributes of each zone 100 in the food production environment and is classified into, for example, a contaminated work zone, a semi-clean work zone, and a clean work zone.

The space information indicates space conditions in the food production environment and includes, for example, temperature, humidity, air purification level, and air pressure.

The cleaning information indicates the cleaning status of the food production environment when the cleaning work has been performed by a worker, and includes, for example, the worker who performed the cleaning work, the elapsed time after the cleaning work, the cleaning agent used during the cleaning work, and the cleaning method used during the cleaning work.

The worker information indicates the work status of a worker performing work in the food production environment and includes, for example, the flow of movement of the worker.

When a food contamination analysis inspection is conducted by operating test equipment in the laboratory 11 according to the test conditions corresponding to the above-described food production environment information, the training data acquisition unit 500 acquires the test results as the environmental contamination indicator detection information and the hazardous substance occurrence information, and also acquires the test conditions as the food manufacturing environment information, thereby acquiring the training data 13 a.

The machine learning unit 501 inputs multiple sets of the training data 13a to the training model 12a and causes the training model 12a to learn the correlation between the environmental contamination indicator information and the hazardous substance information included in the training data 13a to generate a learned training model 12a.

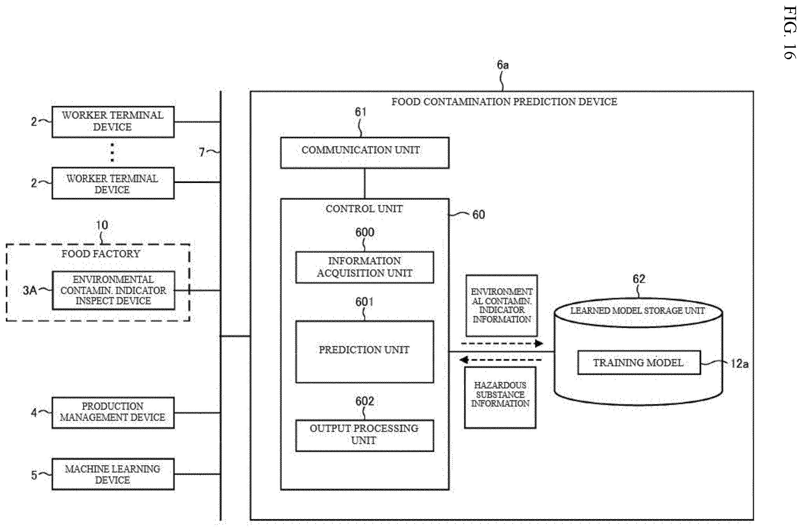

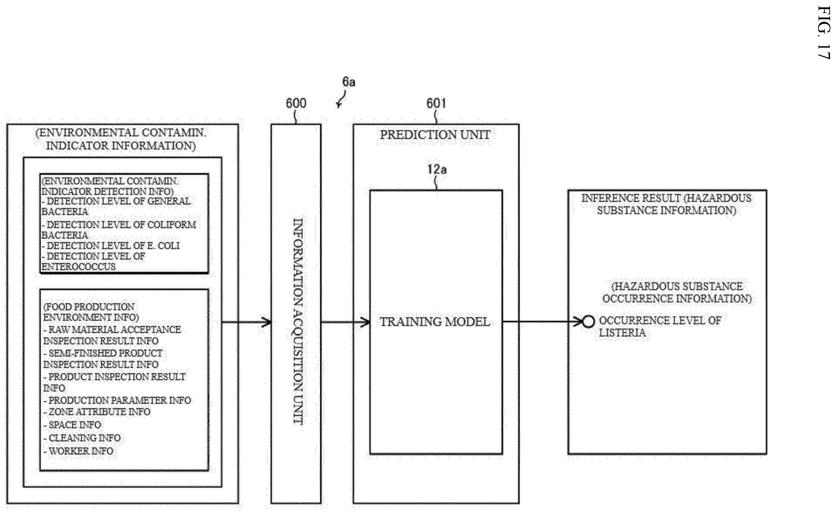

FIG. 16 is a block diagram illustrating an example of the food contamination prediction device 6a according to the second embodiment. FIG. 17 is a functional diagram illustrating an example of the food contamination prediction device 6a according to the second embodiment.

The information acquisition unit 600 acquires the environmental contamination indicator information including the environmental contamination indicator detection information and the food production environment information at the predicted point. At this time, the information acquisition unit 600 refers to the information registered in the process management database 40, the zone management database 41, and the worker management database 42 to acquire the food production environment information. Note that, the food production environment information may include at least one of the raw material acceptance inspection result information, the semi-finished product inspection result information, the product inspection result information, the production parameter information, the zone attribute information, the space information, the cleaning information, and the worker information, or may include information other than the above as long as the information indicates the status of the food production environment.

As described above, the prediction unit 601 predicts the occurrence status of the hazardous substance at the predicted point on the basis of the hazardous substance information output when the environmental contamination indicator information at the predicted point obtained by the information acquisition unit 600 is input to the training model 12 as input data.

As described above, with the food contamination prediction device 6a and the food contamination prediction method according to the present embodiment, since the occurrence status of the hazardous substance is predicted on the basis of the hazardous substance information output when the environmental contamination indicator information, including the environmental contamination indicator detection information and the food production environment information, is input to the training model 12a, the occurrence status of the hazardous substance can be easily predicted without directly inspecting the hazardous substance. In this case, since the food production environment information is reflected, the occurrence status of the hazardous substance can be predicted with higher accuracy.

### Third Embodiment

A third embodiment differs from the first embodiment in that the environmental contamination indicator information includes detection statuses of the environmental contamination indicator at a plurality of points in a predetermined region and the hazardous substance information includes an occurrence distribution of the hazardous substances in that region. The following description will focus on differences to the first embodiment, namely, a machine learning device 5b and a food contamination prediction device 6b according to the third embodiment.

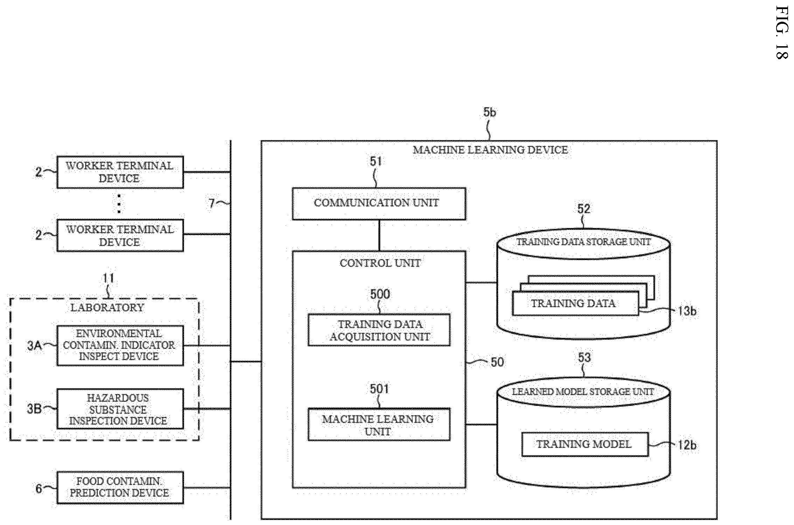

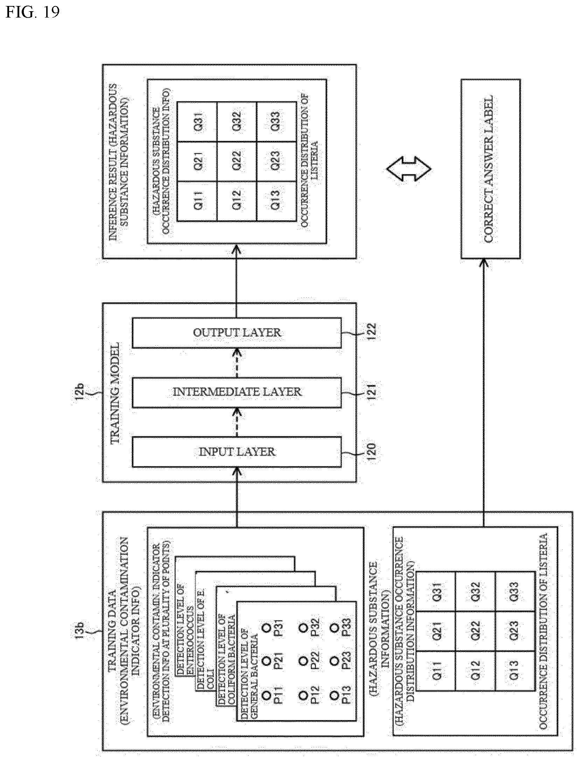

FIG. 18 is a block diagram illustrating an example of the machine learning device 5b according to the third embodiment. FIG. 19 is a diagram illustrating an example of a training model 12b and training data 13b according to the third embodiment. The training data 13b is used in machine learning for the training model 12b.

The environmental contamination indicator information of the training data 13b includes the environmental contamination indicator detection information at a plurality of training points within a training area. The environmental contamination indicator information illustrated in FIG. 19 includes the environmental contamination indicator detection information based on the inspection results obtained by the environmental contamination indicator inspection device 3A inspecting for the environmental contamination indicators at nine locations P11 to P33.

The hazardous substance information of the training data 13b includes the hazardous substance occurrence distribution information indicating an occurrence distribution status of the hazardous substances in the training area. For example, when the training area is divided into meshes of a predetermined size, the hazardous substance occurrence distribution information indicates the occurrence status of the hazardous substances in each mesh. The hazardous substance information illustrated in FIG. 19 includes the hazardous substance occurrence distribution information when the study area is divided into nine meshes Q11 to Q33. The hazardous substance occurrence distribution information is based on results of inspection by the hazardous substance inspection device 3B when the hazardous substances were inspected at points (e.g., the center of each mesh) in each of the nine meshes. Note that, when the occurrence status of the hazardous substance is expressed as an occurrence level in a range from 0 to 1, for example, the occurrence distribution status of the hazardous substance is expressed on a map corresponding to the training area using shading or colors corresponding to the occurrence level value of each mesh.

When a food contamination analysis test is conducted, the training data acquisition unit 500 acquires the training data 13b by acquiring the detection status of the environmental contamination indicator by the environmental contamination indicator inspection device 3A at a plurality of points (training points) in the laboratory 11 and the occurrence status of the hazardous substance by the hazardous substance inspection device 3B at multiple points in the laboratory 11 (points corresponding to each mesh, which may be the same or different points from the study points).

The machine learning unit 501 inputs multiple sets of the training data 13b to the training model 12b and causes the training model 12b to learn the correlation between the environmental contamination indicator information and the hazardous substance information in the training data 13b, thereby generating a learned training model 12b.

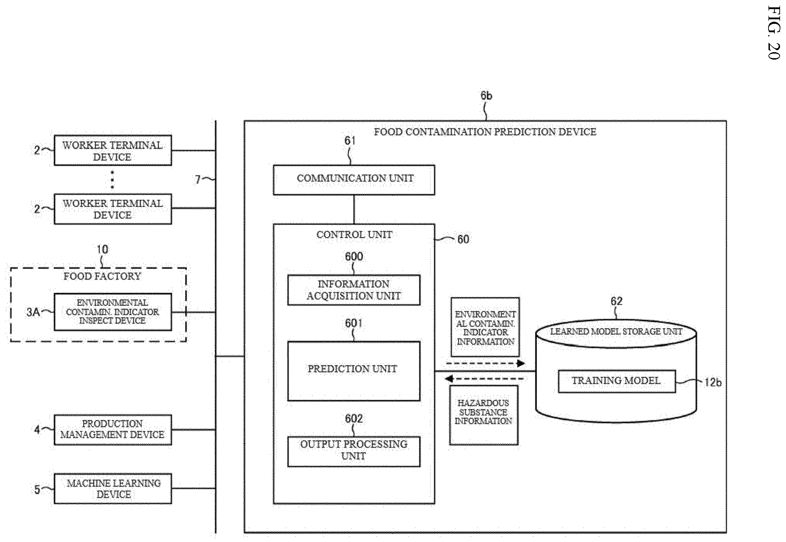

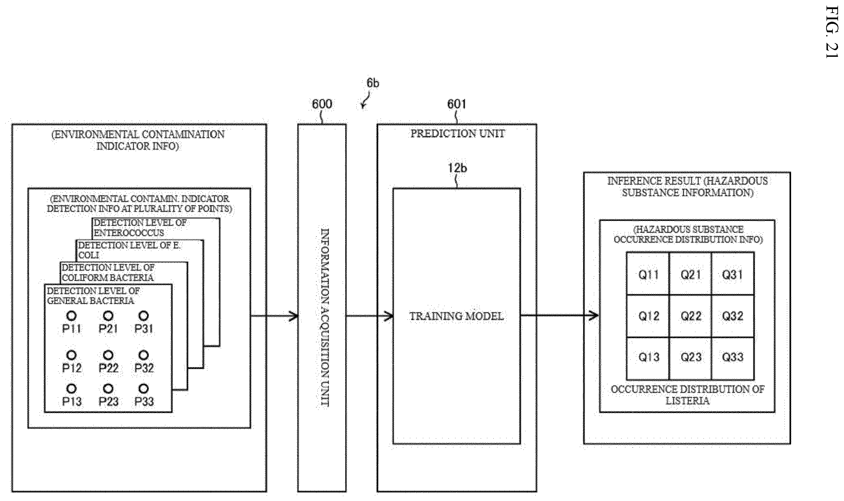

FIG. 20 is a block diagram illustrating an example of the food contamination prediction device 6b according to the third embodiment. FIG. 21 is a functional diagram illustrating an example of the food contamination prediction device 6b according to the third embodiment.

The information acquisition unit 600 acquires the environmental contamination indicator information including the environmental contamination indicator detection information at multiple prediction points within the food factory 10 (prediction area). Note that, the prediction area may be the entire food factory 10, one or more zones 100, or a portion of one or more zones 100.

As described above, the prediction unit 601 predicts the occurrence distribution status of the hazardous substance in the food factory 10 on the basis of the hazardous substance information output when the environmental contamination indicator information in the food factory 10 acquired by the information acquisition unit 600 is input to the training model 12b as input data. The occurrence distribution status of the hazardous substance can be used as, for example, a biohazard map by assigning shading or colors corresponding to the occurrence level in each mesh.

As described above, with the food contamination prediction device 6b and the food contamination prediction method according to the present embodiment, since the occurrence distribution status of the hazardous substance is predicted on the basis of the hazardous substance information output when the environmental contamination indicator information including the environmental contamination indicator detection information is input to the training model 12b as input data, the occurrence distribution status of the hazardous substance can be easily predicted without directly inspecting the hazardous substance.

### Fourth Embodiment

A fourth embodiment is different from the first embodiment in that, similar to the third embodiment, the environmental contamination indicator information includes the detection status of the environmental contamination indicator in a plurality of points in a predetermined region and the hazardous substance information includes a contamination source of the hazardous substance in that region. The following description will focus on differences to the first and third embodiments, namely, a machine learning device 5c and a food contamination prediction device 6c according to the fourth embodiment.

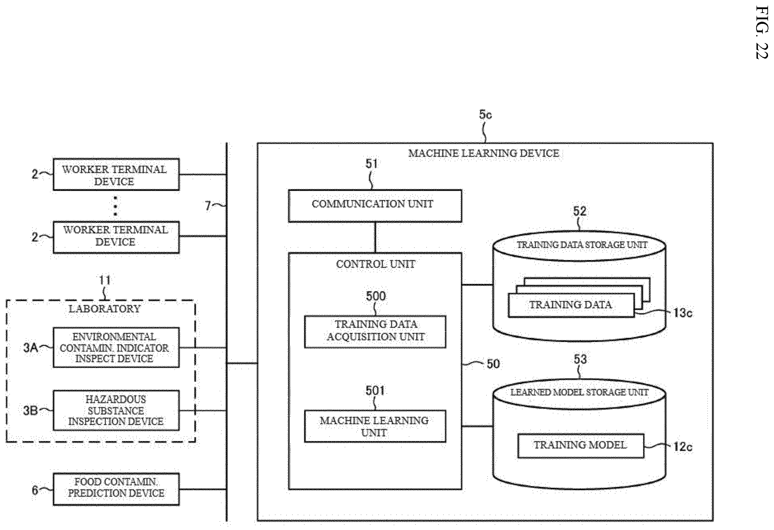

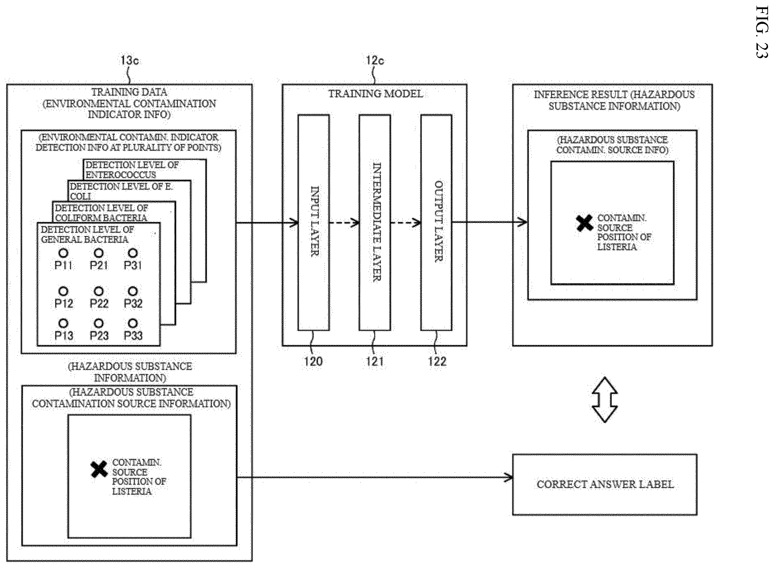

FIG. 22 is a block diagram illustrating an example of the machine learning device 5c according to the fourth embodiment. FIG. 23 is a diagram illustrating an example of a training model 12c and training data 13c according to the fourth embodiment. The training data 13c is used in machine learning for the training model 12c.

As in the third embodiment, the environmental contamination indicator information of the training data 13c includes the environmental contamination indicator detection information at a plurality of learning points within the training area.

The hazardous substance information of the training data 13c includes hazardous substance contamination source information indicating a contamination source of the hazardous substance in the training area. As illustrated in FIG. 23, the hazardous substance contamination source information may indicate the location of the contamination source in the training area, or may indicate a region of a predetermined size where the contamination source is present.

When a food contamination analysis test is conducted, the training data acquisition unit 500 acquires the training data 13c by acquiring the detection status of the environmental contamination indicator by the environmental contamination indicator inspection device 3A at a plurality of points (training points) in the laboratory 11 and the occurrence status of the hazardous substance by the hazardous substance inspection device 3B at a plurality of points in the laboratory 11 and identifying the position of the contamination source of the hazardous substance.

The machine learning unit 501 inputs multiple sets of the training data 13c to the training model 12c and causes the training model 12c to learn the correlation between the environmental contamination indicator information and the hazardous substance information in the training data 13c to generate a learned training model 12c.

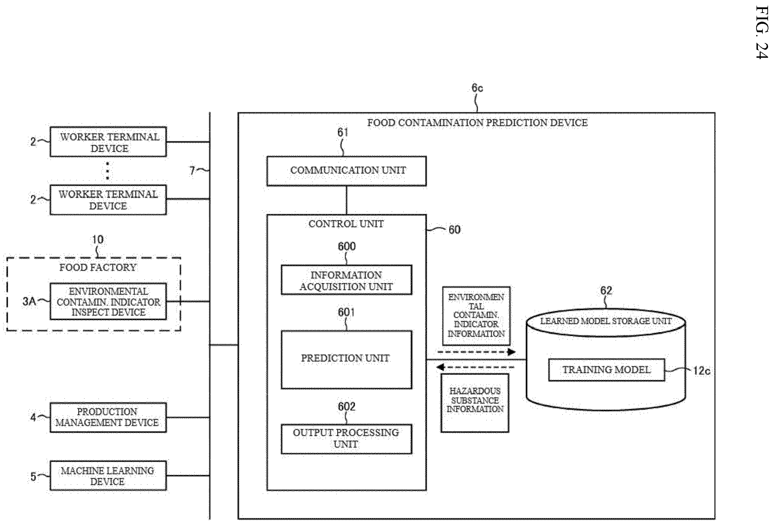

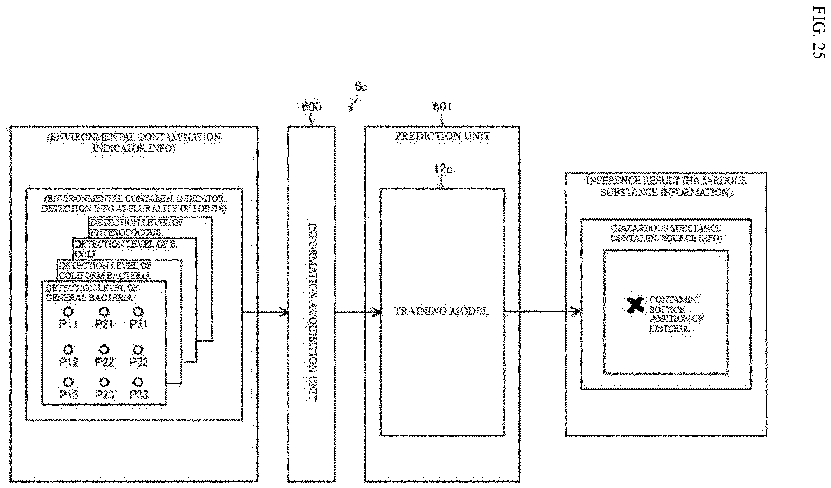

FIG. 24 is a block diagram illustrating an example of the food contamination prediction device 6c according to the fourth embodiment. FIG. 25 is a functional diagram illustrating an example of the food contamination prediction device 6c according to the fourth embodiment.

The information acquisition unit 600 acquires the environmental contamination indicator information including the environmental contamination indicator detection information at a plurality of predicted points in the food factory 10 (predicted region). The prediction area may be the entire food factory 10, one or more zones 100, or a portion of one or more zones 100.

As described above, the prediction unit 601 predicts the contamination source of the hazardous substance in the food factory 10 on the basis of the hazardous substance information output when the environmental contamination indicator information in the food factory 10 acquired by the information acquisition unit 600 is input to the training model 12c as input data.

As described above, with the food contamination prediction device 6c and the food contamination prediction method according to the present embodiment, since the contamination source of the hazardous substance is predicted on the basis of the hazardous substance information output when the environmental contamination indicator information including the environmental contamination indicator detection information is input to the training model 12c as input data, the contamination source of the hazardous substance can be easily predicted without directly inspecting the hazardous substance.

### Other Embodiments

The present invention is not limited to the above-described embodiments, and may be modified within a range that does not depart from the gist of the invention. Any and all modifications are included in the technical scope of the present invention.

The above-described embodiments may be combined as desired. For example, the third embodiment or the fourth embodiment may be combined with the second embodiment such that the environmental contamination indicator information includes the environmental contamination indicator detection information and the food production environment information. Additionally, the third embodiment may be combined with the fourth embodiment such that the hazardous substance information includes the hazardous substance occurrence distribution information and the hazardous substance contamination source information. In these cases, the training model and/or the data configuration of the training data may be changed as required.

In the above-described embodiments, the food contamination prediction device 6, 6a to 6c is described as predicting the hazardous substance information at a point in time when the environmental contamination indicator information is obtained (current time point). However, the food contamination prediction device 6, 6a to 6c may also predict the hazardous substance information at a future point in time (future time point) relative to the current time point. In this case, the food contamination prediction device 6, 6a to 6c may predict the hazardous substance information at the current time point and the hazardous substance information at the future time point, or may predict changes over time in the hazardous substance information by predicting the hazardous substance information at multiple future time points (e.g., hourly). In these cases, the training model and the data configuration of the training data may be changed as required.

In the above-described embodiments, the production management device 4, the machine learning device 5, 5a to 5c, and the food contamination prediction device 6, 6a to 6c are described as separate devices, but these three devices may be configured as a single device, or two of the three devices may be configured as a single device. Alternatively, at least one of the machine learning device 5, 5a to 5c and the food contamination prediction device 6, 6a to 6c may be incorporated into the worker terminal device 2.

In the above-described embodiments, the training model 12, 12a to 12c subjected to machine learning by the machine learning unit 501 is described as using a neural network, but another machine learning model may be used. Examples of the other machine learning method include tree types such as decision trees and regression trees, ensemble learning such as bagging and boosting, neural network types (including deep learning) such as recurrent neural networks, convolutional neural networks, LSTM, clustering types such as hierarchical clustering, non-hierarchical clustering, k-nearest neighbor method, k-means method, multivariate analysis such as principal component analysis, factor analysis, logistic regression, and a support vector machine.

### Machine Learning Program and Food Contamination Prediction Program

The present invention can be provided in the form of a program (machine learning program) for causing the computer 900 to function as the units provided by the machine learning device 5, or a program (machine learning program) for causing the computer 900 to execute each process provided by the machine learning method. The present invention can also be provided in the form of a program for causing a computer 900 to function as each part provided by the food contamination prediction device 6 (food contamination prediction program), or a program for causing a computer 900 to execute each process provided by the food contamination prediction method according to the above embodiment (food contamination prediction program).

### Inference Device, Inference Method, and Inference Program

The present invention can be provided not only in the form of the food contamination prediction device 6 (food contamination prediction method or food contamination prediction program) of the above-described embodiments but also in the form of an inference device (inference method or inference program) used to infer the hazardous substance information. In this case, the inference device (inference method or inference program) may include a memory and a processor, of which the processor executes a series of processing. The series of processing includes information acquisition processing for acquiring the environmental contamination indicator information (information acquisition process), and inference processing for inferring the occurrence status, occurrence distribution status, or contamination source of the hazardous substance (inference process) once the environmental contamination indicator information is acquired in the information acquisition processing.

By providing the inference device (inference method or inference program) in the form of an inference device (inference method or inference program), application to various devices is easier than when using a food contamination prediction device. It is naturally understood by those skilled in the art that when the inference device (inference method or inference program) infers the hazardous substance information, the inference method implemented by the prediction unit may be applied using the learned training model generated by the machine learning device and machine learning method pertaining to the above embodiment.

### Reference Signs List

1 Food production management system
2 Worker terminal device
3A Environmental contamination indicator inspection device
3B Hazardous substance inspection device
4 Production management device
5, 5a to 5c Machine learning device
6, 6a to 6c Food contamination prediction device
7 Network
10 Food factory
11 Laboratory
12, 12a to 12c Training model
13, 13a to 13c Training data
40 Process management database
41 Zone management database
42 Worker management database
43 Inspection database
50 Control unit
51 Communication unit
52 Training data storage unit
53 Learned model storage unit
60 Control unit
61 Communication unit
62 Learned model storage unit
500 Training data acquisition unit
501 Machine learning unit
600 Information acquisition unit
601 Prediction unit
602 Output processing unit

## Claims

1. A food contamination prediction device for predicting an occurrence status of a hazardous substance that may cause a food incident, the food contamination prediction device comprising:
an information acquisition unit configured to acquire, at a prediction point, environmental contamination indicator information including a detection status of an environmental contamination indicator different to the hazardous substance; and
a prediction unit configured to predict the occurrence status of the hazardous substance at the prediction point on the basis of the hazardous substance information, the hazardous substance information being output when the environmental contamination indicator information acquired by the information acquisition unit is input to a training model learned by machine learning to learn a correlation between the environmental contamination indicator information including the detection status of the environmental contamination indicator at a training point and hazardous substance information including the occurrence status of the hazardous substance at the training point.

2. The food contamination prediction device according to claim 1, wherein
the information acquisition unit acquires the environmental contamination indicator information including a detection status of the environmental contamination indicator at a plurality of the prediction points in a prediction region, and
the prediction unit predicts an occurrence distribution status of the hazardous substance in the prediction region on the basis of the hazardous substance information, the hazardous substance information being output when the environmental contamination indicator information acquired by the information acquisition unit is input to a training model learned by machine learning to learn a correlation between the environmental contamination indicator information including the detection status of the environmental contamination indicator at a plurality of the training points in a training region and the hazardous substance information including the occurrence distribution status of the hazardous substance in the training region.

3. The food contamination prediction device according to claim 1 or 2, wherein
the information acquisition unit acquires the environmental contamination indicator information including a detection status of the environmental contamination indicator at a plurality of the prediction points in a prediction region, and
the prediction unit predicts a contamination source of the hazardous substance in the prediction region on the basis of the hazardous substance information, the hazardous substance information being output when the environmental contamination indicator information acquired by the information acquisition unit is input to a training model learned by machine learning to learn a correlation between the environmental contamination indicator information including the detection status of the environmental contamination indicator at a plurality of the training points in a training region and the hazardous substance information including the contamination source of the hazardous substance in the training region.

4. The food contamination prediction device according to any one of claims 1 to 3, wherein the environmental contamination indicator information includes food production environment information indicating a status of a food production environment including the prediction point or the training point.

5. The food contamination prediction device according to any one of claims 4,
wherein the environmental contamination indicator information includes, as the food production environment information, cleaning information indicating a cleaning status of the food production environment.

6. The food contamination prediction device according to claim 4 or 5, wherein the environmental contamination indicator information includes, as the food production environment information, at least one of raw material acceptance inspection result information indicating an acceptance inspection result for raw materials used in a food production process performed in the food production environment, product inspection result information indicating an inspection result of a product produced by the food production process, and a semi-finished inspection result information indicating an inspection result of a semi-finished product produced by the food production process.

7. The food contamination prediction device according to any one of claims 4 to 6, wherein the environmental contamination indicator information includes, as the food production environment information, production parameter information indicating a production parameter of the food production process performed in the food production environment.

8. The food contamination prediction device according to any one of claims 4 to 7, wherein the environmental contamination indicator information includes, as the food production environment information, space information indicating a space status in the food production environment.

9. The food contamination prediction device according to any one of claims 4 to 8, wherein the environmental contamination indicator information includes, as the food production environment information, zone attribute information indicating an attribute of a zone in the food production environment.

10. The food contamination prediction device according to any one of claims 4 to 9, wherein the environmental contamination indicator information includes, as the food production environment information, worker information indicating a work status of a worker who works in the food production environment.

11. An inference device including a memory and a processor and that infers an occurrence status of a hazardous substance that may cause a food incident, the processor performing:
information acquisition process of acquiring, at a prediction point, environmental contamination indicator information including a detection status of an environmental contamination indicator different to the hazardous substance; and
inference process of, when the environmental contamination indicator information is acquired in the information acquisition process, inferring the occurrence status of the hazardous substance at the prediction point.

12. A machine learning device that generates a training model for predicting an occurrence status of a hazardous substance that may cause a food incident, the machine learning device comprising:
a training data storage unit configured to store multiple sets of training data, the training data including the environmental contamination indicator information including a detection status of an environmental contamination indicator different to the hazardous substance at a training point, and hazardous substance information including an occurrence status of the hazardous substance at the training point;
a machine learning unit configured to input the multiple sets of training data to a training model to cause the training model to learn a correlation between the environmental contamination indicator information and the hazardous substance information;
and a learned model storage unit configured to store the training model caused to learn the correlation by the machine learning unit.

13. A food contamination prediction method for predicting an occurrence status of a hazardous substance that may cause a food incident, the method comprising:
an information acquisition process of acquiring, at a prediction point, environmental contamination indicator information including a detection status of an environmental contamination indicator different to the hazardous substance; and
a prediction process of predicting the occurrence status of the hazardous substance at the prediction point on the basis of the hazardous substance information, the hazardous substance information being output when the environmental contamination indicator information acquired by the information acquisition process is input to a training model learned by machine learning to learn a correlation between the environmental contamination indicator information including the detection status of the environmental contamination indicator at a training point and hazardous substance information including the occurrence status of the hazardous substance at the training point.

14. An inference method for inferring an occurrence status of a hazardous substance that may cause a food incident, the method being executed by an inference device including a memory and a processor, the processor performing:
an information acquisition process of acquiring, at a prediction point, environmental contamination indicator information including a detection status of an environmental contamination indicator different to the hazardous substance; and
an inference process of, when the environmental contamination indicator information is acquired in the information acquisition processing, inferring the occurrence status of the hazardous substance at the prediction point.

15. A machine learning method for generating a training model for predicting an occurrence status of a hazardous substance that may cause a food incident, the method comprising:
a training data storage process of storing multiple sets of training data, the training data including the environmental contamination indicator information including a detection status of an environmental contamination indicator different to the hazardous substance at a training point, and hazardous substance information including an occurrence status of the hazardous substance at the training point;
a machine learning process of inputting the multiple sets of training data to a training model to cause the training model to learn a correlation between the environmental contamination indicator information and the hazardous substance information;
and a learned model storage process of storing the training model caused to learn the correlation by the machine learning unit.
